# DEMANDE DE BREVET EUROPEEN

(11) **EP 4 356 998 A1**
(43) Date de publication de la demande: **24.04.2024**
(21) Numéro de dépôt: 23204489.1
(22) Date de dépôt: 18.10.2023
(51) Int. Cl.: B01D 5/00, B01D 8/00, G01N 30/00, G01N 33/00, G01N 27/403, G01N 1/38, G01N 1/40, G01N 30/72

(54) **SYSTÈME COMPRENANT AU MOINS UN PIÈGE À FROID ET UN MÉLANGEUR DE SOLVANTS RELIÉ À L'ENTRÉE DU PIÈGE FROID, INSTALLATION DE MESURE DE GAZ CONTENANT LE SYSTÈME, APPLICATION À L'ANALYSE DE GAZ OPERANDO GÉNÉRÉS PAR UNE BATTERIE AU LITHIUM**

(30) Priorité: 19.10.2022 FR 2210831
(71) Demandeur: Commissariat à l'énergie atomique et aux énergies alternatives, 75015 Paris (FR)
(72) Inventeur: PROFATILOVA, Irina, 38054 GRENOBLE CEDEX 09 (FR); PICHARDO, Mélanie, 38054 GRENOBLE CEDEX 09 (FR)
(74) Mandataire: Cabinet Nony

(57) **Abrégé**

Système comprenant au moins un piège à froid et un mélangeur de solvants relié à l'entrée du piège froid, Installation de mesure de gaz contenant le système, Application à l'analyse de gaz opérande générés par une batterie au lithium.

L'invention concerne un système comprenant :
- au moins un piège à froid (8) comprenant une entrée et une sortie de gaz susceptibles de contenir des vapeurs de solvants ;
- au moins un mélangeur de solvants (11) comprenant une entrée et une sortie de gaz susceptibles de contenir des vapeurs de solvants, la sortie du mélangeur étant reliée à l'entrée du piège à froid, le mélangeur comprenant en son sein au moins un média poreux imprégné d'au moins un solvant destiné à se mélanger avec au moins une partie des vapeurs de solvants provenant de l'entrée du mélangeur.

## Description

### Domaine technique

La présente invention concerne de manière générale toute installation mettant en oeuvre au moins un piège à froid pour condenser des vapeurs.

Elle a trait plus particulièrement aux installations d'analyse de gaz dans lesquelles au moins un piège à froid est agencé en amont du dispositif de mesure proprement dit, de sorte à collecter/piéger le maximum de solvants volatils, afin d'améliorer la mesure des gaz en aval.

Bien que décrite plus spécifiquement en relation avec une application d'analyse de gaz, en conditions opérande, d'une batterie au lithium, les applications envisagées pour l'invention concernent toutes les installations d'analyse de gaz mettant en oeuvre au moins un piège à froid.

De manière plus générale, l'invention peut être mise en oeuvre dans toute installation dans laquelle on utilise un piège à froid qui présente un risque de colmatage dû à la formation d'une quantité trop importante de produits solides lors du fonctionnement. En particulier, une installation d'analyse de gaz dans laquelle un piège à froid est agencé en amont du dispositif de mesure proprement dit.

Par « piège à froid » on entend ici et dans le cadre de l'invention, le sens usuel à savoir un dispositif est un appareil qui condense toutes les vapeurs sauf les gaz permanents en liquide ou solide.

Par « gaz permanent » on entend un gaz qui ne peut pas se liquéfier à une température de fonctionnement d'un piège à froid et par simple augmentation de pression.

### Technique antérieure

La compréhension des mécanismes de fonctionnement ou de dégradation des performances des matériaux de batteries au lithium fait désormais partie des axes incontournables de la conception et développement des batteries.

Cette compréhension a été rendue possible par la mise en oeuvre de techniques d'analyse des matériaux.

Les méthodes implémentées en conditions dites in situ ou « *operando* », qui analysent le matériau d'un accumulateur/d'une batterie en fonctionnement, sont privilégiées afin d'accéder à la dynamique des processus, c'est-à-dire au changement de structure et de composition du matériau et de l'interface électrode/électrolyte en fonction de l'état de charge.

Parmi ces méthodes, on peut citer les mesures de Spectrométrie de Masse Electrochimique En ligne (OEMS, acronyme anglo-saxon pour «*Online Electrochemical Mass Spectrometry* ») qui permettent une identification et quantification des gaz produits pendant le fonctionnement par au moins une cellule électrochimique formée d'une cathode, d'une anode et d'un électrolyte intercalé entre la cathode et l'anode : [1].

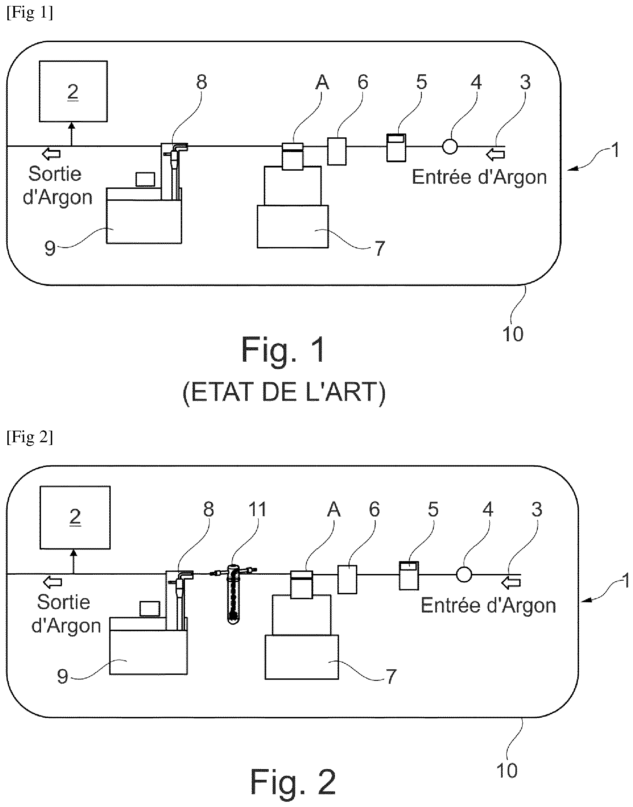

On a représenté à la figure 1 une installation 1 d'analyse de gaz d'une batterie ou accumulateur A au lithium à cellule électrochimique unique ou à empilement de cellules électrochimiques mettant en oeuvre un spectromètre de masse 2, selon l'état de l'art.

Pour l'analyse, les cellules électrochimiques sont réalisées avec un électrolyte contenant différentes combinaisons de solvants dont du carbonate d'éthylène (EC), du carbonate de propylène (PC), du carbonate de diméthyle (DMC), du carbonate de diéthyle (DEC) et du carbonate de méthyle et d'éthyle (EMC) avec un sel LiPF₆ 1M. L'électrolyte peut contenir une certaine quantité d'additifs, typiquement à moins de 10% en masse ou volume dans l'électrolyte, qui ne perturbent généralement pas les mesures par DEMS en raison de leur faible teneur.

Cette installation 1 comprend une unique ligne fluidique 3 dans laquelle de l'argon, de l'hélium ou un autre gaz inerte en tant que gaz porteur/vecteur est injecté en entrée sur la droite de la figure 1.

Sur cette ligne fluidique 3 sont agencés en aval de l'entrée d'argon et en amont de l'accumulateur A, reliés les uns aux autres en série fluidique, un régulateur de pression 4, un régulateur de débit 5 et un barboteur de solvants 6.

Une fonction du barboteur de solvants 6 est de saturer l'argon avec des composants électrolytiques volatils pour empêcher l'électrolyte de sécher dans la ou les cellules électrochimiques de l'accumulateur A pendant la réaction électrochimique puisqu'un test de mesure des gaz peut prendre plusieurs jours.

Un potentiostat et/ou galvanostat 7 est(sont) relié(s) électriquement en parallèle à l'accumulateur A pour faire des cycles de test en maintenant un courant électrique suffisamment constant et/ou une tension suffisamment constante afin de pouvoir évaluer le potentiel électrique de la (des) cellule(s) électrochimique(s) de l'accumulateur A.

Les vapeurs de solvants volatils provenant de la (des) cellule(s) électrochimique(s) de l'accumulateur A et du barboteur 6, tels que le carbonate de diméthyle (DMC), le carbonate de diéthyle (DEC) et le carbonate de méthyle éthyle (EMC) peuvent polluer le spectromètre de masse 2 et masquer les signaux des gaz générés à l'intérieur de la (des) cellule(s).

Par exemple, le CO₂, dont le signal de spectrométrie de masse m/z est égal à 44, est normalement formé par la décomposition de l'électrolyte pendant le cycle de fonctionnement de l'accumulateur A. Cependant, les solvants DMC et EMC donnent également un signal m/z égal à 44 en raison de la fragmentation de DMC et EMC à l'intérieur de la chambre d'analyse du spectromètre de masse 2.

Ainsi, le signal émis par CO₂ peut être masqué si une quantité de solvants DMC et d'EMC trop importante entre dans le spectromètre de masse 2 par rapport à la quantité de CO2 générée par la(les) cellule(s) électrochimique (s). Cela est également le cas de signaux émis par le CO à m/z égal à 28, le C₂H₄ à m/z égal à 26, O₂ à m/z égal à 32.

La publication [2] a proposé l'utilisation d'une membrane spécifique, non perméable aux solvants pour éviter la pollution d'un spectromètre de masse avec les solvants dégagés sous haute tension à une cathode de matériau LiNi_{0.5}Mn_{1.5}O₄ (LNMO).

Dans l'installation selon la figure 1, au lieu d'une membrane spécifique, un piège à froid 8 alimenté en liquide cryogénique par un cryostat 9 est agencé sur la ligne fluidique 3 entre l'accumulateur A et le spectromètre de masse 2.

Ce piège à froid 8 a pour but de collecter un maximum de solvants volatils pour améliorer les mesures de gaz, notamment du CO₂, H₂, CO, C₂H₄, O₂ et le cas échéant d'autres espèces possibles en fonction de la composition de l'électrolyte comme C₂H₆, C₂H₂, CH₂. La température du piège à froid 8 est régulée à une basse température, typiquement à -76°C dans un exemple de fonctionnement de l'installation de la figure 1, afin de garantir un maximum d'efficacité de la collecte.

Pour optimiser la régulation, l'ensemble de la ligne fluidique 3 avec les composants précités de l'installation 1 est situé dans une pièce 10 à atmosphère sèche avec une température régulée à l'intérieur basse pour éviter la formation de glace sur les parois du piège à froid 8, qui peut réduire son efficacité. Typiquement, dans l'exemple de fonctionnement de l'installation de la figure 1, la température intérieure de la pièce sèche 10 est égale à -40 °C.

Comme cela ressort de la publication [3], les différents solvants précédemment énoncés ont des propriétés physiques différentes, dont certaines synthétisées dans le tableau 1 ci-dessous.

**[Tableau 1]**

| Solvant | Pression de vapeur partielle (Pa) à la température T considérée (K) | T° du liquidus (°C) |
|---|---|---|
| DMC | 1381,7 (T = 271.64K) | 5,2 |
| EMC | 4300 (T = 273K) | -53,6 |
| DEC | 272 (T = 273K) | -74,8 |
| EC | 0,25 (T = 273K) | 38,2 |

Pour le DMC il ressort de ce tableau 1 que même si sa température du liquidus du DMC est de 5,2 °C, sa pression de vapeur est élevée.

Dans une installation comme celle de la figure 1, compte tenu de la valeur de débit de gaz porteur/vecteur et d'un volume intérieur de piège à froid limité, il est nécessaire d'utiliser des températures beaucoup plus basses pour assurer le captage de la plus grande partie des vapeurs de solvants volatils. Typiquement dans l'exemple de fonctionnement de l'installation 1, le débit d'argon est compris entre 0,3 et 5ml/min, notamment égal à 2,2 ml/min et le volume intérieur du piège à froid 8 est de l'ordre de 30 ml.

De plus, le DMC est généralement en mélange avec d'autres carbonates organiques en sortie d'accumulateur A. Il est nécessaire d'avoir un volume et un diamètre limités du piège à froid 8 si l'on souhaite un temps de réponse relativement court, c'est-à-dire un temps entre l'instant où les gaz sont générés dans la(les) cellule(s) électrochimique(s) de l'accumulateur A et leur détection par le spectromètre de masse 2. A titre d'exemple, le temps de réponse peut être compris entre 15 et 40 min, en fonction de la configuration du test.

Or, dans les conditions précitées, les inventeurs ont constaté qu'un problème de colmatage du piège à froid 8 peut apparaître du fait de la solidification du DMC, de l'EMC et de leurs mélanges éventuels au sein même du un piège froid après une certaine période de fonctionnement de l'installation. Typiquement dans l'exemple de fonctionnement de l'installation 1, cette période peut être au bout de 45h d'utilisation.

Il existe donc un besoin pour améliorer les installations de mesure de gaz générés par un dispositif électrochimique, tel qu'une cellule électrochimique d'un accumulateur/batterie, notamment afin d'éviter à tout le moins réduire le colmatage d'un piège à froid dû à la solidification de solvants volatils présents dans le mélange de gaz entrant dans le piège à froid.

De manière plus générale, il existe un besoin pour améliorer toute installation dans laquelle au moins un piège à gaz est mis en oeuvre, notamment afin d'éviter à tout le moins réduire son colmatage induit par une solidification de solvants en son sein.

Le but de l'invention est de répondre au moins en partie à ce besoin.

### Exposé de l'invention

Pour ce faire, l'invention a tout d'abord pour objet un système comprenant :
- au moins un piège à froid comprenant une entrée et une sortie de gaz susceptibles de contenir des vapeurs de solvants ;
- au moins un mélangeur de solvants comprenant une entrée et une sortie de gaz susceptibles de contenir des vapeurs de solvants, la sortie du mélangeur étant reliée à l'entrée du piège à froid, le mélangeur comprenant en son sein au moins un média poreux imprégné d'au moins un solvant destiné à se mélanger avec au moins une partie des vapeurs de solvants provenant de l'entrée du mélangeur.

Par « un média poreux imprégné d'au moins un solvant », on entend ici et dans le cadre de l'invention le sens usuel, à savoir un média dont l'intérieur du corps est pénétré du solvant, qui se diffuse à l'intérieur du média. En particulier, le média poreux absorbe le solvant mais ne l'adsorbe pas : il s'agit d'une absorption et non d'une adsorption.

De préférence, l'au moins un solvant imprégnant le média poreux est destiné à se mélanger avec l'au moins une partie des vapeurs de solvants provenant de l'entrée du mélangeur de sorte à former une solution eutectique ou pré-eutectique.

L'au moins un solvant comporte de préférence du DEC, optionnellement uniquement du DEC. L'au moins un solvant peut également comporter du carbonate de propylène et/ou du carbonate de méthyle et d'éthyle et/ou du carbonate de dipropyle, en combinaison ou non avec du DEC.

Selon un mode de réalisation avantageux, le mélangeur de solvants comprend deux tubes logés l'un dans l'autre par une jonction J assurant l'étanchéité du volume intérieur délimité entre eux et l'extérieur, le tube extérieur formant le corps du mélangeur étant borgne et est en forme de L avec une branche débouchante sur l'extérieur par une ouverture formant la sortie des gaz, le tube intérieur étant de forme coudée et percée à ses deux extrémités dont celle coudée par une ouverture formant l'entrée des gaz et de vapeurs de solvants et l'autre extrémité par une ouverture qui débouche sur le fond borgne du tube extérieur, le média poreux étant logé et s'étendant sur au moins une partie de la hauteur du tube intérieur.

Avantageusement, le média poreux est choisi parmi au moins un matériau à base de fibres non tissées, un film en polymère, un matériau à base de fibres de carbone.

Les fibres non tissées sont de préférence à base de nylon, coton, polyesters, verre ou un mélange de ceux-ci.

Le film polymère est de préférence choisi parmi le polyéthylène (PE), polypropylène (PP), poly(tétrafluoroéthylène) (PTFE), poly(chlorure de vinyle) (PVC)), fibres d'aramide (polyamide aromatique), le difluorure de polyvinylidène (PVDF) et leurs mélanges, le cas échéant en étant revêtu d'un ou plusieurs revêtements céramiques.

Selon une variante de réalisation avantageuse, le mélange de solvants comprend une pastille frittée poreuse agencée en aval du média poreux.

Ainsi, l'invention consiste essentiellement à agencer en amont d'un piège à froid un mélangeur de solvants avec un média poreux déjà imprégné d'au moins un solvant qui par ses propriétés physiques va pouvoir se mélanger avec des vapeurs de solvants qui entrent dans le mélangeur.

Grâce à ce mélangeur, on peut collecter/piéger le maximum de solvants volatils, et éviter à tout le moins fortement réduire le colmatage du piège à froid en aval qui va donc pouvoir garder son efficacité sur une période plus longue.

L'invention concerne également une installation d'analyse des gaz comprenant un système tel que décrit précédemment. L'installation peut notamment comporter un dispositif d'analyse de gaz, en particulier un spectromètre de masse.

Au final, l'invention apporte de nombreux avantages, notamment par à la solution selon la figure 1, parmi lesquels on peut citer :
- simplicité de mise en oeuvre du mélangeur de solvants sans modifier le reste d'une installation existante,
- suppression du colmatage du piège à froid,
- efficacité du piège à froid sur une période de fonctionnement plus longue,
- amélioration de la mesure des gaz notamment par spectrométrie de masse en aval du piège à froid.

D'autre avantages et caractéristiques ressortiront mieux à la lecture de la description détaillée, faite à titre illustratif et non limitatif, en référence aux figures suivantes.

### Brève description des dessins

[Fig 1] la figure 1 est une vue schématique d'une installation de mesure de gaz générés par une batterie au lithium mettant un spectromètre de masse OEMS selon l'état de l'art.
[Fig 2] la figure 2 est une vue schématique d'une installation de mesure de gaz générés par une batterie au lithium mettant un spectromètre de masse OEMS selon l'invention.
[Fig 3] la figure 3 est une vue en coupe longitudinale d'un pièce à froid utilisé dans l'installation de la figure 2 et fixé dans un vase dans lequel un liquide cryogénique circule.
[Fig 4] la figure 4 est une vue en coupe longitudinale montant les deux pièces principales formant le piège à froid de la figure 3.
[Fig 5] la figure 5 est une vue en perspective et transparence d'un mélangeur de solvants selon un mode de l'invention, destiné à être utilisé en amont du piège à froid dans l'installation de la figure 2.
[Fig 6] la figure 6 est une vue en coupe longitudinale montant les deux pièces principales formant une variante d'un mélangeur à solvants.
[Fig 7A], [Fig 7B] les figures 7A et 7B sont des diagrammes de phase pour les mélanges de solvants binaires DMC/DEC d'une part et EMC/DEC présents au sein d'un accumulateur A et le barboteur depuis le début d'un essai en tant que partie de l'électrolyte à base de carbonate organique introduit dans l'accumulateur, testé dans l'installation de la figure 2.
[Fig 8] la figure 8 est une vue en perspective et en transparence d'un mélangeur de solvants selon une variante de l'invention, destiné à être utilisé en amont du piège à froid dans l'installation de la figure 2.
[Fig 9] la figure 9 illustre sous forme de courbes les quantifications des gaz mesurés par le spectromètre de masse de l'installation de la figure 1 selon l'état de l'art.
[Fig 10] la figure 10 illustre sous forme de courbes les quantifications des gaz mesurés par le spectromètre de masse de l'installation de la figure 2 selon l'invention.

### Description détaillée

Par souci de clarté, les mêmes éléments sont désignés par les mêmes références numériques selon l'état de l'art et selon l'invention.

On précise que dans l'ensemble de la demande, les termes « entrée », « sortie », « amont », «aval » sont à comprendre en relation avec le sens de la circulation des gaz et vapeurs considérés au sein d'une installation selon l'état de l'art et selon l'invention.

La figure 1 a déjà été commentée en détail en préambule. Elle ne le sera donc pas ci-après.

Pour éviter le colmatage d'un piège à froid 8 de l'installation 1 selon la figure 1, qui est induit par une solidification de solvants en son sein, les inventeurs ont pensé à agencer un mélangeur de solvants 11 comme illustré à la figure 2.

Ainsi, le mélangeur de solvants 11 est agencé entre l'accumulateur A dont on cherche à mesurer les gaz émis par son électrochimie en fonctionnement, et le piège à froid 8.

Autrement dit, la sortie du mélangeur de solvants 11 est reliée directement par la ligne fluidique unique 3 à l'entrée du piège à froid 8.

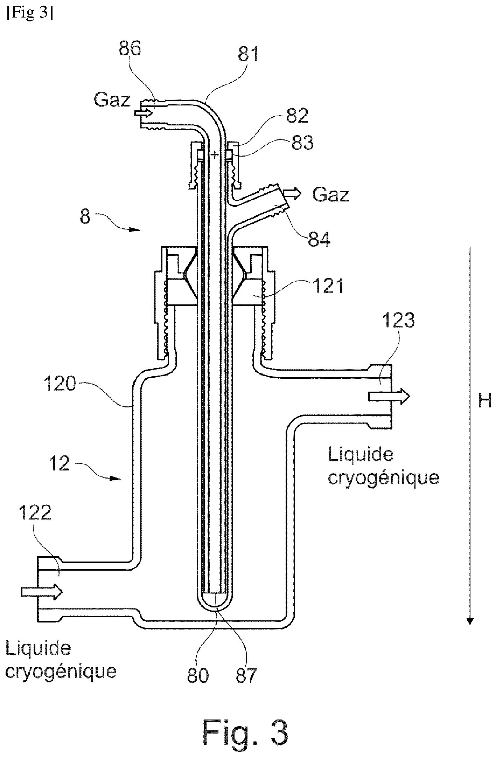

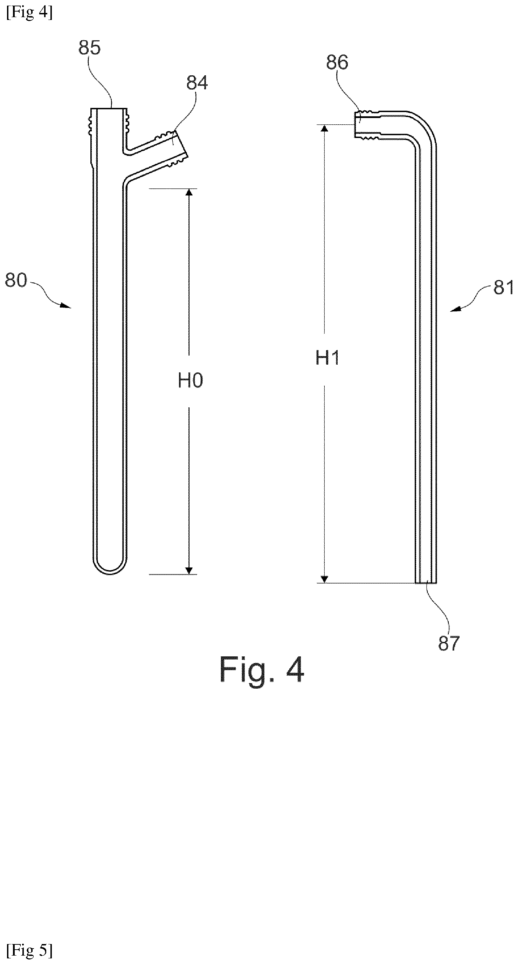

Un exemple de piège à froid 8 qui peut être utilisé dans l'installation de la figure 2 est montré aux figures 3 et 4.

Ce piège à froid 8 comprend deux tubes 80, 81, montés l'un dans l'autre avec un bouchon d'étanchéité 82 vissé sur le tube extérieur 80 et un joint 83 interposé assurant l'étanchéité du volume intérieur délimité entre eux et l'extérieur. A titre d'exemple, les tubes intérieur et extérieur du piège à froid 8 sont en verre borosilicate.

Plus précisément, le tube extérieur 80 forme le corps du piège : ce tube 80 est borgne et est en forme de Y dont l'une des branches est débouchante sur l'extérieur par une ouverture 84 formant la sortie des gaz du piège à froid 8 et l'autre des branches est débouchante sur l'extérieur par une ouverture 85 formant le passage du tube intérieur 81. A titre d'exemple, la hauteur H0 entre le fond borgne et la branche percée de l'ouverture 84 est égale à 165 mm.

Le tube intérieur 81 de forme coudée est percée à ses deux extrémités dont celle coudée par une ouverture 86 formant l'entrée des gaz du piège à froid 8 et l'autre extrémité par une ouverture 87 qui débouche sur le fond borgne du tube extérieur 80. A titre d'exemple, la hauteur H1 entre les deux ouvertures 86, 87 est égale à 215 mm.

Comme montré sur la figure 3, ce piège à froid 8 est monté de manière étanche dans un dispositif à liquide cryogénique 12. Plus précisément ce dispositif 12 comprend un récipient 120 dans lequel une majeure partie rectiligne des tubes 80, 81 du piège à froid 8 est logée en étant fixée de manière étanche dans un bouchon d'étanchéité 121 lui-même vissée sur le récipient 120. A titre d'exemple, la hauteur H du piège à froid qui est fixée dans le récipient est égale à 140mm.

Ce récipient 120 comprend deux ouvertures débouchantes 122, 123 formant respectivement l'entrée et la sortie d'un liquide cryogénique provenant d'un cryostat 9. Ainsi, en fonctionnement, la partie du piège à froid 8 immergée dans le liquide cryogénique circulant dans le récipient 120 est à une température souhaitée et contrôlée pour condenser des vapeurs de solvants volatils au sein du tube intérieur 80. Typiquement, la température cryogénique au sein du récipient 120 peut être égale à -76 °C.

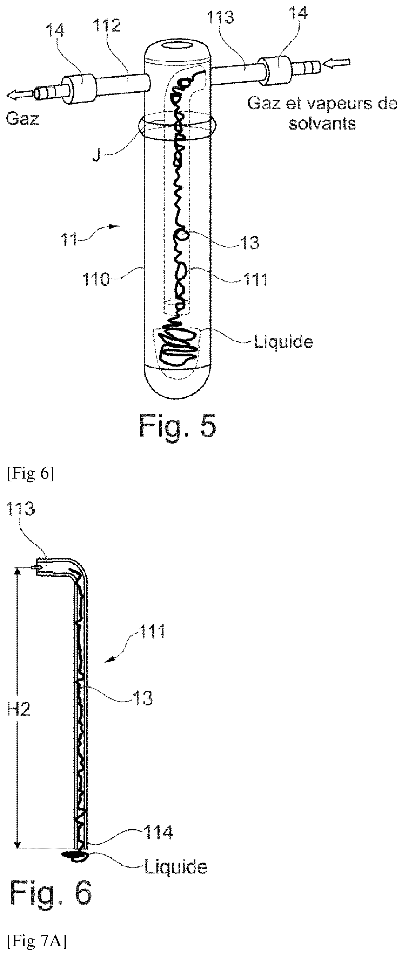

Un mode de réalisation d'un mélangeur à solvants selon l'invention qui peut être utilisé dans l'installation de la figure 2 est montré aux figures 5 et 6.

Ce mélangeur de solvants 11 comprend deux tubes 110, 111 logés l'un dans l'autre par une jonction J assurant l'étanchéité du volume intérieur délimité entre eux et l'extérieur. A titre d'exemple, les tubes intérieur et extérieur du mélangeur sont en verre borosilicate et la jonction J est un cordon de verre.

Plus précisément, le tube extérieur 110 forme le corps du mélangeur: ce tube 110 est borgne et est en forme de L avec la petite branche débouchante sur l'extérieur par une ouverture 112 formant la sortie des gaz du mélangeur 11. A titre d'exemple, la hauteur du tube 110 est comprise entre 150 et 190mm.

Le tube intérieur 111 de forme coudée est percée à ses deux extrémités dont celle coudée par une ouverture 113 formant l'entrée des gaz et de vapeurs de solvants du mélangeur 11 et l'autre extrémité par une ouverture 114 qui débouche sur le fond borgne du tube extérieur 110. A titre d'exemple, la hauteur H2 entre les deux ouvertures 113, 114 est égale à 215mm. Avantageusement, on peut prévoir que le tube intérieur 81 du piège à froid 8 et le tube intérieur 111 du mélangeur 11 soient identiques.

Un bouchon de connexion fluidique 14 peut être fixé de préférence par vissage sur l'ouverture 112 et/ou l'ouverture 113 pour la connexion à la ligne fluidique 3.

Le mélangeur 11 comprend en outre un média poreux 13 logé à l'intérieur du tube intérieur 111 sur au moins une partie de sa hauteur, et qui est imprégné par au moins un solvant par exemple de DEC. A titre d'exemple le matériau du média poreux 13 est une membrane microporeuse monocouche en PP de 25µm d'épaisseur, notamment commercialisée sous la dénomination commerciale Celgard^{®} 2400.

Pour réaliser l'imprégnation du média poreux, du DEC liquide peut être introduit par l'ouverture 113 avant la mise en place du mélangeur 11 sur la ligne fluidique 3 de l'installation. Le DEC sous forme liquide aura tendance à diminuer partiellement au cours de la vie de l'installation. Du fait de la porosité du média 13, une partie du DEC sous forme liquide est maintenue dans la partie non immergée du média 13 par les forces capillaires.

Dans la configuration de la figure 6, le média 13 peut déborder légèrement du tube intérieur 111 et peut venir au contact du solvant au fond du tube, pour assurer une imprégnation continue.

Le fonctionnement du mélangeur à solvants 11 est le suivant : le gaz et les vapeurs de solvants (DEC et/ou EMC et/ou DMC) en sortie de l'accumulateur A entrent dans le tube intérieur 111 et passent ainsi à travers le média poreux 13 imprégné de DEC. Cela provoque un mélange des vapeurs de DMC et/ou d'EMC avec le DEC qui est imprégné dans le média poreux 13.

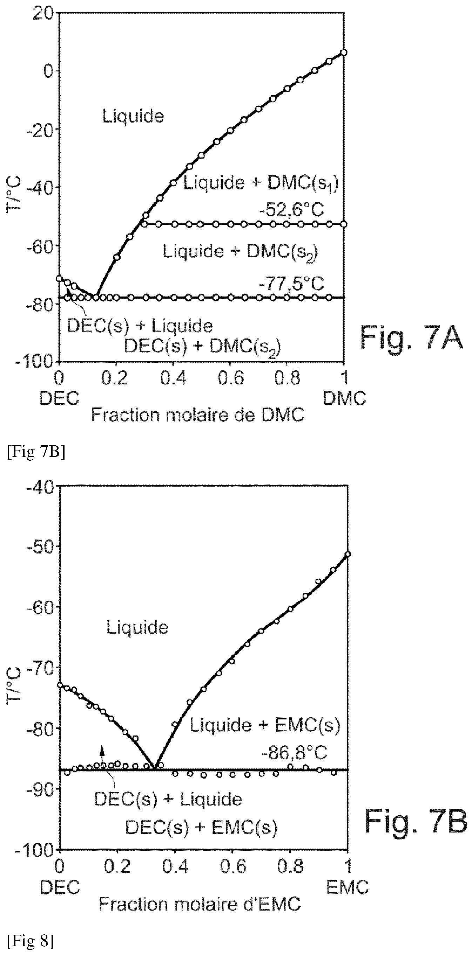

En effet, les vapeurs de solvant DMC et EMC sont tous deux capables de former une solution eutectique ou pré-eutectique, du fait que le DEC présente une température de liquidus/solidus beaucoup plus faible que celle du DMC ou de l'EMC pur, comme illustré par les diagrammes de phase des figures 7A et 7B, extraits de la publication [4].

De plus, comme déjà indiqué dans le tableau 1, le DEC a une pression de vapeur inférieure à celle du DMC et à celle de l'EMC.

Le mélange obtenu d'EMC et/ou de DMC avec du DEC liquide ne forme pas de dépôt solide, car il reste majoritairement liquide, dans les conditions de l'expérience.

Ainsi, on supprime le risque de colmatage du piège à froid 8 dans les conditions de fonctionnement sous liquide cryogénique, typiquement à -76 °C.

Une fois passés le piège à froid 8, les gaz sont analysés par le spectromètre de masse 2 de l'installation 1.

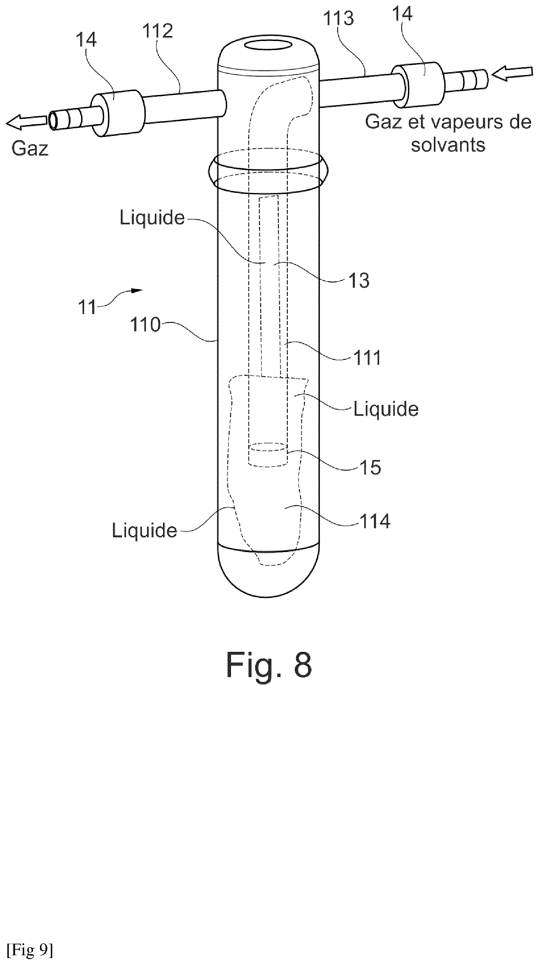

Une variante de réalisation du mélangeur à solvants 11 est illustrée à la figure 8 : une pastille frittée poreuse 15 est ajoutée en bas du média poreux 13 à l'intérieur du tube intérieur 111. Cette pastille frittée poreuse 15 induit une diffusion efficace des gaz et crée une surface de contact maximale entre les vapeurs de solvant qui passent et le DEC pour former un mélange. Cette bonne diffusion des gaz permet de faire plonger l'extrémité 114 du tube 111 muni du média poreux 13 dans le solvant et ainsi s'affranchir de la formation de grosses bulles susceptibles de perturber la mesure.

Dans la conception du mélangeur de solvants, on cherche à éviter la formation de grosses bulles en son sein. Si le tube intérieur 111 est immergé dans le DEC, une grosse bulle de gaz peut se former avant d'être évacué par la sortie du mélangeur vers le piège à froid. Cela peut donc perturber la pression à l'intérieur de la ligne fluidique 3 et donc affecter les mesures de spectrométrie de masse. Par conséquent, soit on dimensionne le tube intérieur 111 de sorte qu'il ne touche pas le DEC liquide, avec le média poreux à l'intérieur qui est imprégné de DEC pour établir une zone de contact pour différentes vapeurs de solvants, soit le tube intérieur 111 qui comprend une pastille frittée poreuse 15 est immergée dans le DEC liquide de sorte à générer de petites bulles de gaz sans perturbation importante de la pression et en assurant également le mélange de vapeurs de solvants.

Les inventeurs ont procédé à des essais comparatifs avec (installation selon la figure 2) et sans (installation selon la figure 1) le mélangeur de solvants 11 selon l'invention.

Les mesures effectués par le spectromètre de masse 2 ont été effectuées avec une même cellule électrochimique dont la cathode est en NMC, l'anode en Si-C, et un mélange EC/DMC/EMC/FEC (3:3:3:1) + 2,0 % en poids de carbonate de vinylène VC + 0,5 % en poids de LiTFSi + 1,0 % en poids d'acétate de trifluoroéthyle2,2,2 (TFA) et d'électrolyte LiPF6 1 M. Ce mélange a été introduit au début des essais et non formé pendant une réaction électrochimique.

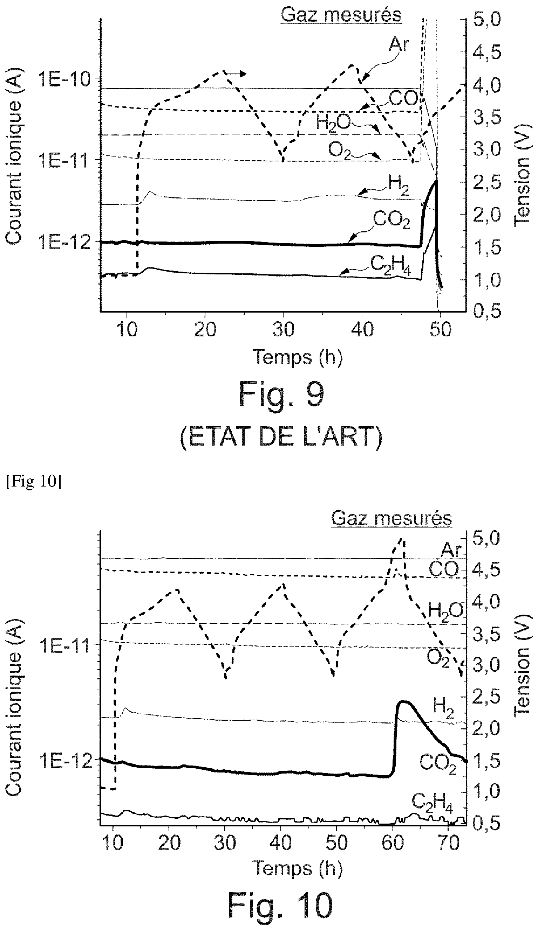

Les profils de tension pour cette cellule qui a été cyclée à un cycle C/10 avec les résultats d'analyse de gaz obtenus par le spectromètre de masse 2 sans le mélangeur de solvant et avec le mélangeur de solvant 11 sont montrés respectivement aux figures 9 et 10. On précise que ces résultats ont été obtenus par un spectromètre de masse commercialisé sous la dénomination commerciale « The Pfeiffer Omnistar GSD 320 mass spectrometer (MS) ».

Il ressort de ces figures 9 et 10 que la durée du fonctionnement de l'installation avec des résultats fiables a pu être prolongée de 47h à 73h grâce à l'ajout du mélangeur de solvant 11 selon l'invention. Son efficacité pour éviter le colmatage du piège à froid 8 en aval est donc prouvée.

En présence du mélangeur de solvant 11, l'essai a été arrêté à 73h, comme prévu sans aucun signe de colmatage du piège à froid 8.

A contrario, comme cela ressort de la figure 9, le colmatage du piège à froid à 47 h a bloqué un passage de gaz normal et l'analyse des gaz générés par la cellule électrochimique n'a plus possible.

Les résultats présentés sont largement reproductibles.

D'autres variantes et améliorations peuvent être envisagées sans pour autant sortir du cadre de l'invention.

Par exemple, d'autres matériaux sont possibles pour le piège à froid et le mélangeur de solvant, comme le PTFE, l'acier inoxydable.

### Références citées

[1]: B.Berkes et al. "Online Continuons Flow Differential Electrochemical Mass Spectrometry with a Realistic Battery Setup for High-Precision, Long-Term Cycling Tests". Anal Chem. 2015 Jun 16;87(12):5878-83.
[2]: Z. Jusys et al, "A novel DEMS approach for studying gas évolution at battery-type electrode/|electrolyte interfaces: High-voltage LiNi0.5Mn1.504 cathode in ethylene and dimethyl carbonate electrolytes", Electrochimica Acta, Volume 314, 2019, 188-201.
[3]: Michael S. Ding, "Liquid-Solid Phase Equilibria and Thermodynamic Modeling for Binary Organic Carbonates", Journal of Chemical & Engineering Data 2004 49 (2), 276-282.
[4]: Ding, Michael et al. (2001). 036. "Liquid/Solid Phase Diagrams of Binary Carbonates for Lithium Batteries", Part II. Journal of The Electrochemical Society. 148. A299-304. 10.1149/1.1353568

## Revendications

1. Système comprenant :
- au moins un piège à froid (8) comprenant une entrée et une sortie de gaz susceptibles de contenir des vapeurs de solvants ;
- au moins un mélangeur de solvants (11) comprenant une entrée et une sortie de gaz susceptibles de contenir des vapeurs de solvants, la sortie du mélangeur étant reliée à l'entrée du piège à froid, le mélangeur comprenant en son sein au moins un média poreux imprégné d'au moins un solvant destiné à se mélanger avec au moins une partie des vapeurs de solvants provenant de l'entrée du mélangeur.

2. Système selon la revendication 1, le mélangeur de solvants (11) comprenant deux tubes (110, 111) logés l'un dans l'autre par une jonction (J) assurant l'étanchéité du volume intérieur délimité entre eux et l'extérieur, le tube extérieur (110) formant le corps du mélangeur étant borgne et est en forme de L avec une branche débouchante sur l'extérieur par une ouverture (112) formant la sortie des gaz, le tube intérieur (111) étant de forme coudée et percée à ses deux extrémités dont celle coudée par une ouverture (113) formant l'entrée des gaz et de vapeurs de solvants et l'autre extrémité par une ouverture (114) qui débouche sur le fond borgne du tube extérieur (110), le média poreux (13) étant logé et s'étendant sur au moins une partie de la hauteur du tube intérieur (111).

3. Système selon la revendication 1 ou 2, le média poreux étant choisi parmi au moins un matériau à base de fibres non tissées, un film en polymère, un matériau à base de fibres de carbone.

4. Système selon la revendication 3, les fibres non tissées étant à base de nylon, coton, polyesters, verre ou un mélange de ceux-ci.

5. Système selon la revendication 3, le film polymère étant choisi parmi le polyéthylène (PE), polypropylène (PP), poly(tétrafluoroéthylène) (PTFE), poly(chlorure de vinyle) (PVC)), fibres d'aramide (polyamide aromatique), le difluorure de polyvinylidène (PVDF) et leurs mélanges, le cas échéant en étant revêtu d'un ou plusieurs revêtements céramiques.

6. Système selon l'une des revendications précédentes, le mélangeur de solvants comprenant une pastille frittée poreuse agencée en aval du média poreux.

7. Installation d'analyse des gaz comprenant un système selon l'une des revendications précédentes.

8. Installation selon la revendication 7 pour la mesure des gaz par spectrométrie de masse d'au moins une cellule électrochimique d'une batterie au lithium, le média poreux étant imprégné du solvant DEC.
